# EUROPEAN PATENT APPLICATION

(11) **EP 2 260 863 A1**
(43) Date of publication of application: **15.12.2010**
(21) Application number: 09725208.4
(22) Date of filing: 27.03.2009
(51) Int. Cl.: A61K 39/395, A61K 31/711, A61K 48/00, A61P 31/04, A61P 31/06, A61P 43/00, C07K 16/12, C12N 15/02, C12P 21/08

(54) **PROPHYLACTIC/THERAPEUTIC AGENT FOR INFECTIOUS DISEASE**

(30) Priority: 27.03.2008 JP 2008083652
(71) Applicant: Takara Bio, Inc., Otsu-shi Shiga 520-2193 (JP)
(72) Inventor: SANO, Mutsumi, Otsu-shi Shiga 520-2193 (JP); AMEMOTO, Mayumi, Otsu-shi Shiga 520-2193 (JP); DODO, Satsuki, Otsu-shi Shiga 520-2193 (JP); KAWANO, Yasuhiro, Otsu-shi Shiga 520-2193 (JP); KATO, Ikunoshin, Otsu-shi Shiga 520-2193 (JP)
(74) Representative: Schnappauf, Georg
(86) International application number: PCT/JP2009/056265
(87) International publication number: WO 2009/119794

(57) **Abstract**

Disclosed is a prophylactic or therapeutic agent for bacterial infectious diseases, which comprises an antibody capable of binding to a toxin or an antitoxin that constitutes a bacterial toxin-antitoxin system to inhibit the interaction between the toxin and the antitoxin or a gene for the antibody as an active ingredient.

SEQUENCE LISTING

<110>  TAKARA BIO INC.

<120>  Preventive or therapeutic agent for infection

<130>  669056

<150>  JP 2008-083652
<151>  2008-03-27

<160>  14

<170>  PatentIn version 3.1

<210>  1
<211>  111
<212>  PRT
<213>  Escherichia coli

<220>
<223>  Amino acid sequence of E.coli MazF.

<400>  1

Met Val Ser Arg Tyr Val Pro Asp Met Gly Asp Leu Ile Trp Val Asp
1               5                   10                  15

Phe Asp Pro Thr Lys Gly Ser Glu Gln Ala Gly His Arg Pro Ala Val
            20                  25                  30

Val Leu Ser Pro Phe Met Tyr Asn Asn Lys Thr Gly Met Cys Leu Cys
        35                  40                  45

Val Pro Cys Thr Thr Gln Ser Lys Gly Tyr Pro Phe Glu Val Val Leu
    50                  55                  60

Ser Gly Gln Glu Arg Asp Gly Val Ala Leu Ala Asp Gln Val Lys Ser
65                  70                  75                  80

Ile Ala Trp Arg Ala Arg Gly Ala Thr Lys Lys Gly Thr Val Ala Pro
                85                  90                  95

Glu Glu Leu Gln Leu Ile Lys Ala Lys Ile Asn Val Leu Ile Gly
            100                 105                 110

<210>  2
<211>  118
<212>  PRT
<213>  Mycobacterium tuberculosis

<220>
<223>  Amino acid sequence of Mycobacterium tuberclosis MazF.

<400>  2

Met Met Arg Arg Gly Glu Ile Trp Gln Val Asp Leu Asp Pro Ala Arg
1               5                   10                  15

Gly Ser Glu Ala Asn Asn Gln Arg Pro Ala Val Val Val Ser Asn Asp
            20                  25                  30

Arg Ala Asn Ala Thr Ala Thr Arg Leu Gly Arg Gly Val Ile Thr Val
        35                  40                  45

Val Pro Val Thr Ser Asn Ile Ala Lys Val Tyr Pro Phe Gln Val Leu
    50                  55                  60

Leu Ser Ala Thr Thr Thr Gly Leu Gln Val Asp Cys Lys Ala Gln Ala
65                  70                  75                  80

Glu Gln Ile Arg Ser Ile Ala Thr Glu Arg Leu Leu Arg Pro Ile Gly
                85                  90                  95

Arg Val Ser Ala Ala Glu Leu Ala Gln Leu Asp Glu Ala Leu Lys Leu
            100                 105                 110

His Leu Asp Leu Trp Ser
        115

<210>  3
<211>  120
<212>  PRT
<213>  Staphylococcus aureus

<220>
<223>  Amino acid sequence of Staphylococcus aureus MazF (S.aureus).

<400>  3

Met Ile Arg Arg Gly Asp Val Tyr Leu Ala Asp Leu Ser Pro Val Gln
1               5                   10                  15

Gly Ser Glu Gln Gly Gly Val Arg Pro Val Val Ile Ile Gln Asn Asp
            20                  25                  30

Thr Gly Asn Lys Tyr Ser Pro Thr Val Ile Val Ala Ala Ile Thr Gly
        35                  40                  45

Arg Ile Asn Lys Ala Lys Ile Pro Thr His Val Glu Ile Glu Lys Lys
    50                  55                  60

Lys Tyr Lys Leu Asp Lys Asp Ser Val Ile Leu Leu Glu Gln Ile Arg
65                  70                  75                  80

Thr Leu Asp Lys Lys Arg Leu Lys Glu Lys Leu Thr Tyr Leu Ser Asp
                85                  90                  95

Asp Lys Met Lys Glu Val Asp Asn Ala Leu Met Ile Ser Leu Gly Leu
            100                 105                 110

Asn Ala Val Ala His Gln Lys Asn
        115                 120

<210>  4
<211>  82
<212>  PRT
<213>  Escherichia coli

<220>
<223>  Amino acid sequence of E.coli MazE.

<400>  4

Met Ile His Ser Ser Val Lys Arg Trp Gly Asn Ser Pro Ala Val Arg
1               5                   10                  15

Ile Pro Ala Thr Leu Met Gln Ala Leu Asn Leu Asn Ile Asp Asp Glu
            20                  25                  30

Val Lys Ile Asp Leu Val Asp Gly Lys Leu Ile Ile Glu Pro Val Arg
        35                  40                  45

Lys Glu Pro Val Phe Thr Leu Ala Glu Leu Val Asn Asp Ile Thr Pro
    50                  55                  60

Glu Asn Leu His Glu Asn Ile Asp Trp Gly Glu Pro Lys Asp Lys Glu
65                  70                  75                  80

Val Trp

<210>  5
<211>  82
<212>  PRT
<213>  Mycobacterium tuberculosis

<220>
<223>  Amino acid sequence of Mycobacterium tuberclosis MazE.

<400>  5

Met Lys Thr Ala Ile Ser Leu Pro Asp Glu Thr Phe Asp Arg Val Ser
1               5                   10                  15

Arg Arg Ala Ser Glu Leu Gly Met Ser Arg Ser Glu Phe Phe Thr Lys
            20                  25                  30

Ala Ala Gln Arg Tyr Leu His Glu Leu Asp Ala Gln Leu Leu Thr Gly
        35                  40                  45

Gln Ile Asp Arg Ala Leu Glu Ser Ile His Gly Thr Asp Glu Ala Glu
    50                  55                  60

Ala Leu Ala Val Ala Asn Ala Tyr Arg Val Leu Glu Thr Met Asp Asp
65                  70                  75                  80

Glu Trp

<210>  6
<211>  56
<212>  PRT
<213>  Staphylococcus aureus

<220>
<223>  Amino acid sequence of Staphylococcus aureus MazE (S. aureus).

<400>  6

Met Leu Ser Phe Ser Gln Asn Arg Ser His Ser Leu Glu Gln Ser Leu
1               5                   10                  15

Lys Glu Gly Tyr Ser Gln Met Ala Asp Leu Asn Leu Ser Leu Ala Asn
            20                  25                  30

Glu Ala Phe Pro Ile Glu Cys Glu Ala Cys Asp Cys Asn Glu Thr Tyr
        35                  40                  45

Leu Ser Ser Asn Ser Thr Asn Glu
50                  55

<210> 7
<211> 351
<212> DNA
<213> Artificial

<220>
<223> Nucleic acid sequence containing MazF(BCG).

<400> 7
catatggtga ttagtcgtgc cgagatctac tgggctgacc tcgggccgcc atcaggcagt     60

cagccggcga agcgccgccc ggtgctcgta atccagtcag atccgtacaa cgcaagtcgc    120

cttgccactg tgaccgcagc ggtgatcacg tccaatacgg cgctggcggc aatgcccggc    180

aacgtgttct tgcccgcgac cacaacgcga ctgccacgtg actcggtcgt caacgtcacg    240

gcgattgtca cgctcaacaa gactgacctc accgaccgag ttggggaggt gccagcgagc    300

ttgatgcacg aggttgaccg aggacttcgt cgcgtactgg accttctcga g             351

<210> 8
<211> 282
<212> DNA
<213> Artificial

<220>
<223> Nucleic acid sequence containing MazE(BCG).

<400> 8
catatgcacc accaccacca ccacatgaag acagctattt ctctgccgga tgagacgttc     60

gatcgggtat cgcggcgtgc gagtgagctc ggcatgagtc ggtccgagtt cttcacgaag    120

gctgcgcagc gctacctgca cgagctggac gcccaattgc tcacgggcca gatcgacagg    180

gctctagaga gcatccatgg caccgacgaa gcggaggccc tcgccgtggc caacgcatac    240

cgcgtgctag aaaccatgga cgatgagtgg tgataaaagc tt                       282

<210> 9
<211> 372
<212> DNA
<213> Artificial

<220>
<223> Nucleic acid sequence containing MazF (S.aureus).

<400> 9
catatgatta gacgaggaga tgtttattta gcagatttat caccagtaca gggatctgaa     60

caagggggag tcagacctgt agtcataatt caaaatgata ctggtaataa atatagtcct    120

acagttattg ttgcggcaat aactggtagg attaataaag cgaaaatacc gacacatgta    180

gagattgaaa agaaaaagta taaattggat aaagactcag ttatattatt agaacaaatt    240

cgtacacttg ataaaaaacg attgaaagaa aaactgacgt acttatccga tgataaaatg    300

aaagaagtag ataatgcact aatgattagt ttagggctga atgcagtagc tcaccagaaa    360

aattagggat cc                                                        372

<210> 10
<211> 794
<212> DNA

<213>  Artificial

<220>
<223>  Nucleic acid sequence containing MazE (S.aureus).

<400>  10
ccatgggcca ccaccaccac caccacatgg caaacattac cgttttctac aacgaagact      60

tccagggtaa acaggtcgat ctgccgcctg gcaactacac ccgggcccag ttggcggcgc     120

tgggcatcga gaacaacacc atcagctcgg tgaaggtgcc gcctggcgtg aaggctatcc     180

tctaccagaa cgatggtttc gccggcgacc agatcgaagt ggtggccaat gccgaggagt     240

tgggcccgct gaacaacaac gtctccagca tccgggtcat atccgtgccc gtgcagccca     300

ggatggcaaa cattaccgtt ttctacaacg aagacttcca gggtaaacag gtcgatctgc     360

cgcctggcaa ctacacccgg gcccagttgg cggcgctggg catcgagaac aacaccatca     420

gctcggtgaa ggtgccgcct ggcgtgaagg ctatcctcta ccagaacgat ggtttcgccg     480

gcgaccagat cgaagtggtg gccaatgccg aggagttggg cccgctgaac aacaacgtct     540

ccagcatccg ggtcatatcc gtgcccgtgc agcccagggg taccgaaaac ctgtattttc     600

agggagctgc agctcatatg ttatctttta gtcaaaatag aagtcatagc ttagaacaat     660

ctttaaaaga aggatattca caaatggctg atttaaatct ctccctagcg aacgaagctt     720

ttccgataga gtgtgaagca tgcgattgca acgaaacata tttatcttct aattcaacga     780

atgaataagg atcc                                                       794

<210>  11
<211>  30
<212>  RNA
<213>  Artificial

<220>
<223>  Synthetic oligonucleotide MazG30.

<400>  11
uaagaaggag auauacauau gaaucaaauc                                       30

<210>  12
<211>  12
<212>  DNA
<213>  Artificial

<220>
<223>  Nucleic acid sequence of substrate-1. Nucleotides 5 is ribonucleo
       tides-other nucleotides are deoxyribonucleotides.

<400>  12
gatauacata tg                                                          12

<210>  13
<211>  13
<212>  DNA
<213>  Artificial

<220>
<223>  Nucleic acid sequence of substrate-2. Nucleotides 6 is ribonucleo
       tides-other nucleotides are deoxyribonucleotides.

<400>  13
tttctuccta tga                                                         13

<210>  14
<211>  15

<212>  DNA
<213>  Artificial

<220>
<223>  Nucleic acid sequence of substrate-3. Nucleotides 6 is ribonucleo
       tides-other nucleotides are deoxyribonucleotides.

<400>  14
tgactuacat cgaag                                                        15

## Description

### Technical Field

The present invention relates to a functional antibody useful for treating infectious diseases, and a therapeutic agent for the infectious diseases, comprising the functional antibody or a gene therefor as an active ingredient.

### Background Art

Among prokaryotic plasmids, those having the function of post-segregation killing (PSK) to kill a host in which a plasmid has been lost, in order to maintain the plasmid in the host, have been reported. Present in these plasmids is a gene (hereinafter described as TA gene) encoding a toxin-antitoxin system (hereinafter described as TA system; in addition, it may also be referred to as TA module, TA complex or addiction module). While an antitoxin inactivates a toxin by binding to a toxin and forming a complex inside the cell, compared to a toxin, an antitoxin is unstable inside the cell, such that a stable toxin is activated by degradation of the antitoxin by proteases (Non-patent Document 1).

Plural TA systems have been reported so far. These are classified into seven two-component families, CcdA-CcdB, RelB-RelE, ParD-ParE, HigA-HigB, MazE/PemI-MazF/PemK, Phd-Doc and VapB-VapC, and a three-component family, ω-ε-ζ (Non-patent Document 1).

As one of such TA systems is MazE-MazF system, present in many bacterial chromosomes. Inoue et al. have demonstrated that a toxin MazF (ChpAK) is an endoribonuclease that recognizes a specific nucleotide sequence (ACA) in a ribosome-independent manner and cleaves mRNA. Christensen et al. have reported that a toxin RelE is an endoribonuclease that recognizes a specific codon of three bases and cleaves mRNA. In addition, they has reported that the toxins ChpAK, ChpBK and YoeB are also endoribonucleases that cleave mRNA in a codon-dependent manner as well. Aside from Escherichia coli, genes for DR0662 from Deinococcus radiodurans, Mb2014c from Mycobacterium bovis BCG and the like (Patent Document 1) and MazF homolog from Mycobacterium tuberculosis (Non-patent Document 2) have been reported as homologs of MazF. Recently, a homolog of MazF has also be found in Staphylococcus aureus (Non-patent Document 3).

At first, MazEF from Escherichia coli has been reported to result in cell death in Escherichia coli (Non-patent Document 4). However, study results and the like have been shown thereafter that, while overexpression of MazF degrades mRNA to inhibit the translation of protein, and as a result thereof, the colony forming capability of Escherichia coli is decreased, cell growth is recovered by expressing the antitoxin MazE gene. From this, MazF is not currently considered as resulting in cell death in Escherichia coli (Non-patent Document 1).

In spite of this, the possibility that the TA system is useful as a drug discovery target for a novel antibiotic has been pointed out. Screening methods for a substance that inhibits formation of a TA system complex have been disclosed in Patent Documents 2 and 3. In these documents, the possibility is shown, that a substance inhibiting complex formation in the TA system works as an antibiotic suppressing the growth of bacteria; however, any of them are simply shown as mere speculation without any scientific basis. Thus, currently, it is not clear whether inhibition of complex formation in the TA system results in suppression of bacterial growth or cell death or not, and any example of infectious disease conditions treated by, or any evidence supporting that they treatable with, an antibody that inhibits the toxin-antitoxin interaction, has not been shown. Even if a relationship between bacterial growth and TA system complex formation are known, it is unclear whether an antibody binding to a toxin or an antitoxin is actually effective in the treatment of infectious diseases due to these bacteria or not. It is understood that a bacteria has many TA systems. Since it is thought that TA system forms a complex network with other TA systems inside the cell, it is not known whether inhibition of the activity of only a toxin or an antitoxin of a bacteria allows an infectious disease conditions due to the bacteria to be treated or not. Any functional antibody or low molecular weight compound, which is effective in inhibiting toxin-antitoxin interaction in bacteria to treat infectious disease conditions due to the bacteria, has been found.
Patent Document 1: WO 2007/013265 pamphlet
Patent Document 2: Japanese Patent Application Publication No.2007-39398
Patent Document 3: WO 2007/109781 pamphlet
Non-patent Document 1: Nature Reviews Microbiology, Vol. 3, pp. 371-382 (2005)
Non-patent Document 2: The Journal of Biological Chemistry, Vol. 281, pp.18638-18643 (2006)
Non-patent Document 3: Journal of Bacteriology, Vol. 189, pp.8871-8879 (2007)
Non-patent Document 4: Proceedings of National Academy of Sciences USA, Vol. 93, pp.6059-6063 (1996)

### Disclosure of Invention

### Problems to be Solved by the Invention

In recent years, the occurrence of multiple drug-resistant bacteria has been a large problem in the medical field, and the development of a novel therapeutic agent for infectious diseases, which targets a specific cellular function, is needed.
Consequently, the present invention was completed in order to solve the above mentioned problems and provide a novel therapeutic agent for infectious diseases.

### Means of Solving the Problems

The present inventors searched for antibodies reacting specifically with toxins possessed by bacteria, and discovered among them a functional antibody with the specific property, that is, the activity of inhibiting complex formation of an antitoxin and a toxin while maintaining the endoribonuclease activity of the toxin, to suppress bacterial growth or killing bacteria, and found that the antibody with this property, an active fragment of this antibody and genes encoding them are widely useful as therapeutic agents for a infection by each infecting bacteria, and finally completed the present invention.

The present invention relates to:
[1] a prophylactic or therapeutic agent for a bacterial infectious disease comprising, as an active ingredient, an antibody binding to a toxin or an antitoxin constituting a bacterial toxin-antitoxin system to inhibit toxin-antitoxin interaction, or a gene thereof,
[2] the prophylactic or therapeutic agent of [1], in which the antibody is a monoclonal antibody against a toxin or an antitoxin,
[3] the prophylactic or therapeutic agent of [2], in which the antibody is a monoclonal antibody against a toxin or an antitoxin of a bacterium selected from the group consisting of Escherichia coli, Mycobacterium tuberculosis and Staphylococcus,
[4] then prophylactic or therapeutic agent described in any of [1] to [3], in which the toxin-antitoxin system is a toxin-antitoxy system containing a toxin having a sequence-specific endoribonuclease activity,
[5] the prophylactic or therapeutic agent described in any of [1] to [4], in which the antibody is a recombinant antibody,
[6] the prophylactic or therapeutic agent described in any of [1] to [5], in which the antibody is a humanized antibody or a fully human antibody,
[7] the prophylactic or therapeutic agent described in any of [1] to [6], in which the bacterial infectious disease is an infectious disease selected from the group consisting of tuberculosis, MRSA infectious disease and VRE infectious disease, and
[8] an inhibitory agent of weight loss in a bacterial infectious disease containing, as an active ingredient, an antibody binding to a toxin or an antitoxin constituting a bacterial toxin-antitoxin system to inhibit toxin-antitoxin interaction.

### Effect of the Invention

According to the present invention, a prophylactic/therapeutic agent for bacterial infectious diseases is provided, comprising as an active ingredient an antibody or a fragment containing the antigen-binding site of the antigen, specifically binding to a toxin or an antitoxin to inhibit the toxin-antitoxin interaction thereby preventing toxin-antitoxin complex formation, thus showing the effect of suppressing bacterial growth or killing bacteria, or genes encoding them.

### Brief Description of Drawings

Fig. 1 shows the inhibition of interaction in the bacterial TA system according to Example 4 of the present invention;
Fig. 2 shows the inhibition of interaction in the bacterial TA system according to Example 5 of the present invention;
Fig. 3 shows the inhibition of interaction in the bacterial TA system according to Example 6 of the present invention;
Fig. 4 shows the inhibition of interaction in the bacterial TA system according to Example 7 of the present invention;
Fig. 5 shows the inhibition of interaction in the bacterial TA system according to Example 7 of the present invention;
Fig. 6 shows the inhibition of interaction in the bacterial TA system according to Example 7 of -the present invention;
Fig. 7 shows the inhibition of interaction in the bacterial TA system according to Example 7 of the present invention;
Fig. 8 shows the inhibition of interaction in the bacterial TA system according to Example 7 of the present invention;
Fig. 9 shows the growth of bacteria according to Example 8 of the present invention; and
Fig. 10 shows the viable bacterial cell count according to Example 9 of the present invention.

### Best Mode for Carrying Out the Invention

In the present invention, "antibody" means an immunoglobulin and a fragment thereof containing the antigen-binding site, specifically reacting with an immunogen (antigen). In the present invention, an antibody produced by a B cell or a fragment thereof comprising the antigen-binding site can be used. As the fragment containing the antigen-binding site, a heavy chain (H chain, Heavy chain), a light chain (L chain, Light chain), an Fab, an F(ab')₂, a variable region (V_{H}, V_{L}) or a constant region or the like can be used alone or in combination. Generally, there are five kinds of heavy chain, γ chain, µ chain, α chain, δ chain and ε chain, and there are two kinds of light chain, λ chain and κ chain. Each antibody molecule of subclasses IgG, IgM, IgA, IgD and IgE is formed by combination of these molecules. As antibody used in the present invention, monoclonal antibodies derived from human or non-human animal such as mouse, humanized antibodies including chimeric antibodies, fully human antibodies, polyclonal antibodies, multivalent antibodies (for instance, a bivalent antibody), multi-specific antibodies (for instance, a bispecific antibody) or one-chain antibodies (a single chain antibody, scFv), furthermore, antibody-like molecules such as a nano antibody developed from antibody possessed by camels and llamas comprising only an H chain (Nanobody), IgNAR from shark or the like can be given as examples.
An antibody used in the present invention can have the specificity and capability of, while maintaining the activity of the toxin of the target bacteria, recognizing a toxin or an antitoxin and binding thereto to inhibit the toxin-antitoxin interaction.

In the present invention, "toxin" and "antitoxin" mean respectively proteic toxin and antitoxin encoded by the plasmid DNA or chromosomal DNA of bacteria. A toxin is also referred to as "toxin (poison)" and antitoxin is also referred to as "antidote (Antidote)". In a bacterial cell, a toxin forms a stable TA complex with an antitoxin, and thus a toxin does not exert toxicity to the cell; however, in response to various stresses in the cell, when an unstable antitoxin is degraded by a protease, then, the toxin exerts its toxicity to control DNA replication and protein synthesis. The bacterial growth regulation system constituted by a toxin and an antitoxin described above is referred to as a toxin-antitoxin system (TA system) or addiction module. Many TA systems have been reported so far. As TA systems whose function is regulated by the antibody according to the present invention, for instance, the following TA systems can be given as examples, without limiting the present invention in particular.

According to a review by Kenn Gerdes et al. [Nature Reviews Microbiology, Volume 3, pp371-382 (2005)], seven TA system families exist in bacteria. In the review, the locus name encoding each TA system, the toxin name, the antitoxin name, the action of the toxin and the number of loci in the prokaryotic genome and the like, are compiled in Table 1. Among these, the RelE toxin encoded by the relBE locus and the MazF toxin (PemK) encoded by the mazEF (also referred to as ChpA) locus are ribonucleases that degrade mRNA. MazE (PemI) (antitoxin)/MazF (toxin) from Escherichia coli is most analyzed TA system. MazF has been shown by Inoue et al. to cleave specifically an ACA sequence in mRNA in a ribosome-independent manner [Molecular cell, Volume 12, pp913-923 (2003)]. Also in regard to RelE, it has been reported to be an endoribonuclease that recognizes a specific three-base codon in a ribosome-dependent manner and cleaves mRNA. Consequently, the toxicities of RelE and MazF result from degradation of mRNA to suppress translation. Very recently, also regarding VapC-1 toxin of Haemophilus influenzae and HigB toxin of Vibrio cholerae, they have been reported to possess ribonuclease activities that degrade mRNA [Journal of Bacteriology, Volume 189, pp5041-5048 (2007); Molecular Microbiology, Volume 62, pp397-411 (2006)]. Note that, MazF is also present in pathogenic Escherichia coli O-157 strain. In addition, very recently, Escherichia coli MazE-MazF has been reported to be present in vancomycin-resistant enterococcus [Proc Natl Acad Sci USA, Volume 104, pp311-316 (2007)].

Note that, in the present specification, in regard to toxin and antitoxin of the MazF and MazE families derived from bacteria other than Escherichia coli, they may be sometimes represented by adding the name of the bacteria of origin to MazF or MazE. MazF (BCG) and MazE (BCG) mean respectively MazF and MazE derived from Mycobacterium bovis BCG, in addition, MazF (S. aureus) and MazE (S. aureus) mean respectively MazF and MazE derived from Staphylococcus aureus. The amino acid sequences of MazF (BCG) and MazE (BCG) are identical to the amino acid sequences of MazF and MazE derived from Mycobacterium tuberculosis described below.

The amino acid sequences of the MazF family and MazE family are given below as examples.
MazF from Escherichia coli (SEQ ID:1)
MazF from Mycobacterium tuberculosis (SEQ ID:2)
MazF (S. aureus) from Staphylococcus aureus (SEQ ID:3)
MazE from Escherichia coli (SEQ ID:4)
MazE from Mycobacterium tuberculosis (SEQ ID:5)
MazE (S. aureus) from Staphylococcus aureus (SEQ ID:6)

### (I) Antibody of the present invention

As long as the antibody of the present invention is an antibody that has the capability of specifically recognizing and binding to a toxin or an antitoxin constituting the TA system of the target bacteria to inhibit the interaction between the toxin and the corresponding antitoxin, it is not limited by the producing method or the preparing method for the antibody, and the antibody can be obtained according to the general methods. As a preferred aspect of the present invention, an antibody recognizing a toxin having an endoribonuclease activity that cleaves mRNA in a sequence-specific manner, or the antitoxin thereof, is given as an example. In the following, examples wherein the toxin MazF and the antitoxin MazE constituting the MazE-MazF system, which is one TA system from Escherichia coli are described for illustrating the antibody of the present invention.

### (1) Preparation of antigen

As antigen used to obtain an anti-MazE antibody or an anti-MazF antibody, a protein MazE or MazF per se, or a peptide derived from MazE or MazF comprising a partial amino acid sequence of at least three contiguous residues thereof, or, for the purpose of increasing antigenicity, a derivative into which a carrier protein such as helper T cell epitope peptide, albumin or keyhole limpet hemocyanin (KLH) or the like is added, can be used.

When MazE or MazF as-is as an antigen is used, the protein can be directly purified from Escherichia coli cell, and one expressed as a recombinant protein can also be used by cloning a DNA encoding MazE or MazF and using recombinant technology. According to the conventional method, MazE gene or MazF gene can be carried by a plasmid vector appropriate for expression in an appropriate prokaryotic cell host, for instance, Escherichia coli or Bacillus subtilis, allowing MazE or MazF to express in these host cells. For immunizing an animal with antigen, a crude extract fraction containing recombinant MazE or MazF can be used, such as a cell extract or a concentrate thereof. When an immunizing antigen is purified for use, in order to facilitate purification of the expression product, a tag sequence such as a His-Tag or HA-Tag, to an extent that does not change antigenicity, can be added to the C-terminus or N-terminus of the antigenic protein. The expression in Escherichia coli and purification of C-terminally His-Tagged MazE or MazF can be carried out according to the methods described in Molecular cell Volume 12, pp913-923 (2003). Preparation of N-terminally His-Tagged MazE or MazF recombinant protein can also be prepared according to the methods described in the above document. For the purpose of improving the amount of recombinant MazE or MazF expression and the solubility of the protein, MazE or MazF may be expressed as a chimeric protein with Protein S from Myxococcus xanthus, according to the methods described in WO 2007/109697 pamphlet.

### (2) Preparation of anti-MazF antibody or anti-MazE antibody used in the present invention

The polyclonal antibody or monoclonal antibody of the present invention can be obtained respectively according to the methods described in [Methods in Enzymology, Volume 182, pp663-670 (1990)] or [Monoclonal Antibodies: Principles and Practice, Academic Press (1996)] by immunizing an appropriate animal with the antigen prepared in (1). In the case of a polyclonal antibody, the serum of the immunized animal is used in the following screening. From the point of view of specificity to the antigen or the function of the antibody or obtaining the antibody gene, a monoclonal antibody is used preferably. In the case of a monoclonal antibody, a hybridoma obtained by the cell fusion of an antibody-producing cell, which has been prepared from the spleen or lymph node of an immunized animal with a myeloma cell can be used in the following screening.

In regard to an antibody obtained by immunizing an animal other than human, in order to reduce immunogenicity upon administeration to human, a "humanized chimeric antibody" can be prepared, in which the variable region of the animal-derived antibody is bonded to a human-derived constant region (Japanese Patent Application Publication No. H7-194384). In addition, immunogenicity can also be decreased without losing ligand binding ability, by changing the antigenicity of the variable region to human antigenicity by amino acid substitution/humanization (Japanese Patent Application Publication No. H8-280387). In addition, a humanized antibody can be prepared by the method of grafting only the animal antibody (donor)-derived complementarity determining region (CDR) portion to the human-derived antibody (acceptor) (reconstitution). A humanized antibody that maintains the reactivity of the original antibody with a lower antigenicity can also be prepared by matching a portion of amino acids from not only the CDR but also the framework portion with those of the donor (Japanese Patent No. 2828340, Japanese Patent Application Publication No. H11-4694, Japanese Patent Application Publication No. H11-243955).

Furthermore, a fully human monoclonal antibody can also be obtained by immunizing with the MazE or MazF protein described in (1) a transgenic animal such as a mouse or a cow producing human antibodies (Japanese Patent Application Publication No. H10-146194, Japanese Patent Application Publication No. H10-155492, Japanese Patent No. 2938569, Japanese Patent Application Publication No. H11-206387, Japanese Patent Application Publication No. H8-509612, Japanese Patent Application Publication No. H11-505107).

In addition to the methods for obtaining antibodies from an antibody-producing cell from an immunized animal as described above, the monoclonal antibody of the present invention can also be obtained by the method of preparing a gene library of antibodies, using the antibody display technique to select from this library an antibody specific to the antigen MazE or MazF.

As antibody gene library,
(i) an immune library prepared by amplifying the genes for the H chain and L chain of an antibody from an antibody-producing cell from an animal or a human immunized with the immunizing antigen prepared in (1), expressing the product in the Fab or single chain antibody (scFv) format,
(ii) a non-immune (naive) library prepared by amplifying the genes for the H chain and L chain of an antibody from peripheral blood lymphocyte or the like from a non-immuned animal or human, expressing the product in the Fab or single chain antibody (scFv) format, or
(iii) a synthetic library prepared by replacing the complementarity determining region (CDR) of the V gene of a specific antibody molecule of an animal or human with an oligonucleotide encoding a random amino acid sequence in appropriate length,
or the like, can be used.

The most general antibody display technique is the phage display method that presents an antibody in the form of an Fab or an scFv on the outer coat protein (g3p) of a filamentous phage (for instance M13 phage). A phage display library that presents Fab or scFv can be constructed according to the methods described in [Phage Display A Laboratory Manual, Cold Spring Harbor Laboratory Press (2001)]. In addition to the phage display library, a library prepared by the yeast surface display method, the ribosome display method, the animal cell surface display method or the like can also be used.

By digesting the obtained antibody with a appropriate protease, a low molecular weight antibody containing the antigen-binding site can be prepared. Molecular weight reduction of antibodies by protease restricton is a classic, well-known method, and for instance, F(ab')₂ can be prepared by digesting IgG1 with pepsin, and Fab by digesting with papain for the preparation of these low molecular weight antibodies, for instance, ImmunoPure (registered trademark) Fab Preparation Kit manufactured by Pierce or the like can be used.

### (3) Screening for antibody used in the present invention

Measurement of antibody potency in the immunized animal serum or measurement of antibody potency in screening monoclonal antibody-producing hybridoma can be carried out in general by using the methods such as the RIA method, the ELISA method or flow cytometry. For instance, when the ELISA method is used, the immunizing antigen as-is can be used for the antigen to coat the plate, but it is preferred to use a MazF that is different from the immunizing antigen. For instance, a purified (partially purified) MazE or MazF that does not possess a His-Tag is used for antibody potency measurement in case a His-Tagged MazE or MazF is used as the immunizing antigen, or C-terminally His-Tagged MazE or MazF can be used for potency measurement in case an N-terminally His-Tagged MazE or MazF is used as the immunizing antigen, and a hybridoma that produces an antibody specifically reacting with MazE or MazF can be obtained by eliminating the antibodies reacting with- the Tag sequence per se or with the MazE or MazF sequence containing the Tag sequence. In addition, nonspecific antibodies can be eliminated by measuring the potency to a protein that is not related to the target antigen, such as BSA. In the case of a monoclonal antibody, it is possible to select hybridomas that produce various monoclonal antibodies, from clones with high antibody potency to clones with low antibody potency, in the hybridoma screening process.

In addition, sequence information of the cDNA encoding the antibody molecule can be obtained from the mRNA of the obtained hybridoma with appropriate primers. For instance, in case of a hybridoma from mouse, with the total RNA of the hybridoma producing the selected antibody as templates, a first strand synthesis using primers designed for the heavy chain CH1 and light chain CL portions and a reverse transcriptase and thereafter a PCR amplification using a known method [Journal of Immunological Methods, Volume 178, pp241-251 (1995)] can be carried out to clone the DNA encoding the antibody heavy chain and light chain variable sites (VH, VL), and to determine the nucleotide sequences thereof using a DNA sequencer. In this way, a gene encoding an anti-MazE antibody or an antibody fragment containing the antigen-binding site of an anti-MazF antibody can be obtained. By determining the nucleotide sequence of the obtained gene, the amino acid sequence information of the anti-MazE antibody or the antibody fragment containing the antigen-binding site of the anti-MazF antibody can be obtained as well. Based on the obtained sequence of the gene encoding the antibody fragment containing the antigen-binding site, an expression plasmid expressing the complete antibody can be constructed by linking the gene encoding the antibody variable region and the gene encoding the antibody constant region. Furthermore, in addition to a complete antibody, a cDNA can be designed, encoding a low molecular weight antibody containing an antigen-binding site, such as, Fab, single chain antibody (scFv), a dimer of single chain antibody (Diabody), a trimer of single chain antibody (Triabody) or Minibody, and by linking these genes to the downstream of a promoter for expression in an appropriate host, plasmids can be constructed for expression of low molecular weight antibodies containing an antigen-binding site. By expressing these antibody fragment expression plasmids in appropriate hosts such as Escherichia coli, yeast, insect cell or animal cell, the antibodies and low molecular weight antibodies described above can be obtained as recombinant proteins.

An operation referred to as biopanning is carried out in order to obtain an antibody specific to MazF using an antibody display technique such as phage display from an Fab or a single chain antibody (scFv) library containing antibody variable site genes. For this, the operation of selecting from an M13 phage antibody library a phage that binds to MazE or MazF according to well known methods, for instance, the methods described in [Phage Display A Laboratory Manual, Cold Spring Harbor Laboratory Press (2001)] are carried out. Basically, phages displaying antibodies specific to the target antigen MazE or MazF are concentrated by repeating the operation of bringing an M13 phage antibody library into contact with MazE or MazF which has been solid-phased on plastic plate or tube, washing and eliminating unbound nonspecific phages using an appropriate buffer solution, for instance, PBS (phosphate-buffered physiological saline) containing 0.1% Tween20 or the like, then, eluting the bound phages with an acidic solution, for instance, 0.1 M glycine-hydrochloric acid (pH 2.2) or the like, neutralizing, and infecting Escherichia coli for multiplication. As a phagemid vector containing the gene encoding the presented antibody is packaged inside a phage, by determining the nucleic acid nucleotide sequence of the the antibody gene-inserted-region of this phagemid vector, the gene of the selected antibody can be cloned and the sequence information thereof be determined. Antibody V regions having various affinities and epitope specificities can be selected by varying the amount of antigen (MazE or MazF) immobilized on the solid phase, or changing the immobilizing method or the like.

Antibodies binding to MazE or MazF, and not completely inhibiting activity of a toxin can be screened from those selected as antibodies specifically binding to MazE or MazF as described above, by measuring the activity of MazF as a toxin, that is to say, the capability of the antibodies or functional fragments thereof of inhibiting sequence specific ribonuclease activity. In addition, antibodies inhibiting the interaction between the toxin MazF and the antitoxin MazE can be screened by measuring the sequence specific ribonuclease activity in the presence of MazE and MazF as well as the antibodies.

Considering that the strength of the binding activity to MazE or MazF and the strength of the inhibitory activity on the formation of the TA complex MazE-MazF are necessarily not correlated, it is preferred to measure the influence on the toxin activity of MazF and influence on MazE-MazF not only for antibodies with strong binding activities with respect to MazE or MazF, but also for antibodies with not so strong binding activities to MazE or MazF.

The ribonuclease activity of Escherichia coli MazF can be measured according to the methods described in the documents [Molecular cell, Volume 12, pp913-923 (2003); Analytical Biochemistry, Volume 371, pp173-183 (2007)], for instance, with a synthetic oligo RNA or a DNA-RNA chimeric oligonucleotide as a substrate. In addition, a method for measuring the ribonuclease activity of MazF with high sensitivity using a synthetic substrate comprising a DNA-RNA chimeric oligonucleotide containing ribonucleotide residues on the 5' end of the MazF cleaving site by, which is labeled at the 5' end with the fluorescent substance ROX and at the 3' end with the quenching substance Eclipse respectively, is described in WO 2007/109781 pamphlet. In addition, a method for measuring the activity of MazF using a cell-free protein synthesis system is described in Molecular cell, Volume 12, pp913-923 (2003).

Antibodies showing no influence at all on the ribonuclease activity of MazF when the antibodies of the present invention are added to these activity measurement systems, and even if showing an influence, demonstrating an inhibitory effect by up to 50%, preferably by up to 20%, are preferred as antibodies of the present invention.

The activity of MazF is suppressed in the presence of MazE in the above-mentioned MazF ribonuclease activity measurement system. While the ratio between MazE and MazF is not limited in particular, it can be set appropriately in a molar ratio range of 1:10 to 2:1, according to the sensitivity of the assay system. According to the above article, the activity of MazF in the cell-free protein synthesis system is suppressed by an approximately equimolar addition of MazE to MazF. In a system for measuring the ribonuclease activity of MazF with a synthetic oligo RNA as a substrate, an inhibitory effect on the ribonuclease activity of MazF can be observed under conditions where the molar ratio between MazE and MazF is approximately 1:4. By adding the antibody of the present invention to such a MazF activity measurement system in the presence of MazE, the inhibitory effect of the antibody of the present invention on the MazE-MazF interaction can be measured. That is to say, under conditions where the inhibitory effect of MazE on the MazF activity can be observed, by adding the present antibody and selecting such an antibody whereby the inhibitory effect of MazE on the MazF activity is suppressed, the antibody of the present invention, that is to say an antibody having the capability of specifically recognizing and binding to MazE or MazF and inhibiting the interaction between MazF and MazE can be obtained.

In addition to assay systems using ribonuclease activity of MazF as described above, a method for observing directly the dissociation of the TA complex (MazE-MazF complex) is disclosed in WO 2007/109781 pamphlet. That is to say, it is a method in which the toxin or the antitoxin is expressed and purified as a fusion protein with a fluorescent protein such as GFP, a His-Tag is added to the corresponding antitoxin or toxin, a complex between fluorescent protein-toxin/antitoxin and antitoxin/toxin-His-Tag is formed, this complex is immobilized on a nickel-agarose bead or the like, a TA complex formation inhibitor is added to this immobilized complex, and the free fluorescence intensity (fluorescent protein-toxin/antitoxin) is measured. By adding the antibody of the present invention to this measurement system and measuring the free fluorescence intensity, an antibody having the capability of specifically binding to the toxin constituting the TA complex of the present invention and inhibiting the interaction between the toxin and the antitoxin can be selected.

The MazE-MazF complex formation inhibitory activity can also be measured by the following method. MazE or MazF is solid-phased on a container, and after blocking with BSA, milk casein or the like, MazF comprising a Tag sequence if MazE has been solid-phased, or MazE comprising a Tag sequence if MazF has been solid-phased, is added. After incubation at room temperature or 37°C for an appropriate period, the container is washed, and then the MazE or MazF bound to the container surface is detected using an antibody against the Tag sequence added respectively. For this purpose, it is preferred to add, in addition to a Tag sequence for purification (for instance His-Tag sequence), different Tag sequences, for instance, a Myc-tag sequence, a HA-tag sequence or the like to MazF and MazE. In case a chimeric protein of MazE or MazF and Myxococcus xanthus Protein S is used, detection is possible using an antibody against Protein S. In case a MazE-MazF complex formation inhibitory activity is measured by adding the antibody of the present invention to the system that carries out assay for the MazE-MazF interaction on a solid-phased plate of MazE or MazF, the complex formation inhibition activity can be measured by mixing a constant amount of MazE or MazF and antibodies at varying concentrations, adding it respectively to the MazF or MazE solid-phased plate, and detecting MazF or MazE binding to MazE or MazF on the solid phase. MazE-MazF complex formation inhibition activity of the antibody used for the therapeutic agent of the present invention can be measured also by detecting and determining with an antimouse labeled antibody or the like, the amount of antibody competing with, conversely, MazE or MazF at varying concentrations.

In another aspect of the present invention, the formation of the TA system complex can be inhibited by expressing the gene encoding the obtained antibody or a fragment containing the antigen-binding site of the antibody in a target bacteria, that is to say, in a bacteria having a TA system that the antibody or the fragment containing the antigen-binding site of the antibody acts upon. For the expression of the above antibody or fragment thereof containing the antigen-binding site in the bacteria, a appropriate vector containing a gene encoding the above antibody or a fragment thereof under control of a appropriate promoter can be prepared. While the vector is not limited in particular, a plasmid vector, a viral vector, a phage vector, or the like, can be used. It is preferred that the expression of the above gene in a target bacteria is controlled by an expression inducing factor, a gene expression inhibitory factor or the like, that is appropriate to the above promoter.

Similarly to MazE/MazF described above, antibodies that can be used in the present invention can also be obtained for toxins and antitoxins from other TA systems.

### (II) Therapeutic agent for infectious diseases of the present invention

Therapeutic agent of the present invention for infectious diseases is a therapeutic agent for infectious disease comprising as an active ingredient an antibody or a fragment containing the antigen-binding site of the antibody, having the activity of binding to a toxin or an antitoxin of the TA system described in (I) to inhibit the TA system complex formation, or a gene encoding the antibody or the fragment containing the antigen-binding site of the antibody. The therapeutic agent for infectious diseases of the present invention results in growth inhibition, induction of programmed cell death or the like, and, in a bacteria having a TA system that an antibody or a fragment containing the antigen-binding site of the antibody which is the active ingredient acts upon. Therefore, the therapeutic agent of the present invention for infectious diseases is useful in treatment of bacterial infections, and if needed, can also be used in preventing bacterial infections.

As infectious diseases caused by bacteria, for instance, infectious diseases caused by (pathogenic) Escherichia coli, Mycobacterium tuberculosis, Staphylococcus aureus, Listeria (Listeria monocytogenes), Salmonella, Pseudomonas aeruginosa or the like can be cited, and in particular, the present invention is useful for infectious diseases caused by multiple drug-resistant bacteria. TA systems that are homologous to the MazE-MazF system, which is the target of the therapeutic agent of the present invention for infectious diseasse, are known to be present in Mycobacterium tuberculosis [Journal of Biological Chemistry, Volume 281, pp18638-18643 (2006)] and Staphylococcus aureus [Journal of Bacteriology, Volume 189 volume, pp8871-8879 (2007)], in addition to Escherichia coli. Also, recently, it has been shown that the MazE-MazF TA module from Escherichia coli is present on the plasmid carrying the drug resistance gene of vancomycin resistant enterococcus (VRE) [Proceedings of National Academy of Sciences USA, Volume 104, pp311-316 (2007)]. Consequently, the therapeutic agent of the present invention for infectious diseases is effective in the treatment of Escherichia coli, Mycobacterium tuberculosis, Staphylococcus aureus, enterococcus as well as antibioticresistant strains from the same species as these.

The therapeutic agent of the present invention for infectious diseases can be safely administered according to well known means used in general for pharmaceutical preparation, with the antibody of the present invention or a fragment containing the antigen-binding site of the antibody as-is or mixed with a pharmaceutically acceptable carrier as a pharmaceutical preparation such as, for instance, tablet, powdered drug, granule, encapsulated formulation, solution, injectable, suppository or controlled release agent.

The content of the antibody or the fragment containing the antigen-binding site of the antibody in the formulation is 1 to 100 % by weight, preferably 10 to 100%, and more preferably 50 to 100%, of the entire formulation. Although the therapeutic agent of the present invention for infectious diseases differs depending on the subject to be treated, subject organ, symptoms, administration method or the like, and is not limited in particular, in general, administering to the patient (as 60 kg body weight), per day, approximately 5 to 2000 mg, preferably approximately 10 to 1000 mg and more preferably approximately 20 to 500 mg, is preferred. Although the administrating method is selected appropriately and is not limited in particular, venous injection is preferred.

As pharmaceutically acceptable carriers, for instance, diluent, lubricant, binder and disintegration agent in solid formulation, or solvent, dissolution adjuvant, suspending agent, isotonization agent, buffer agent, soothing agent in liquid formulation or the like may be cited. Furthermore, as necessary, the conventional additives such as antiseptic agent, antioxidant, pigment, sweetening agent, adsorbent or wetting agent can be used appropriately in suitable amounts.

The therapeutic agent for infectious disease comprising as an active ingredient the gene encoding the antibody of the present invention or a fragment containing the antigen-binding site of the antibody can be a nucleic acid of the naked type, vector type or the like, and the agent containing the above gene can be formulated in the desired dosage form. Herein, the naked type indicates a nucleic acid having no self-replication function of the nucleic acid or no integration function into a particle (phage or virus) in addition to the expression system of the above gene. A vector usable for the therapeutic agent for infectious diseases of the vector type can be chosen appropriately according to the stability of the gene and the efficiency of gene introduction into bacteria; specifically, a plasmid vector, a viral vector or a phage vector is preferred.

As vectors used for the therapeutic agent of the present invention for bacterial infectious diseases comprising as an active ingredient a gene encoding the antibody or a fragment containing the antigen-binding site of the antibody, a phage vector or a phagemid vector is used preferably. A phage is a virus also referred to as bacteriophage that infects bacteria. A phage vector is one vectorized by modifying a phage genome, containing the total genome of a phage in the vector, and capable alone of forming a phage. On the other hand, phagemid vector is a plasmid vector containing the packaging signal into a phage, and since only a portion of the phage constituent proteins is encoded in the phagemid vector, cannot form a phage alone. For the formation of a phage particle, a helper phage is necessary to provide other phage constituent proteins that are necessary for phage particle formation, such that only co-infection of this helper phage with the phagemid vector allows a phage particle to be formed. By inserting the gene encoding the antibody of the present invention or a fragment containing the antigen-binding site of the antibody into these phage vectors or phagemid vectors, these therapeutic genes can be introduced efficiently into the target bacteria, allowing the antibody of the present invention or the fragment containing the antigen-binding site of the antibody to express inside the target bacteria. The phage used in the present invention is not limited in particular, but, for instance, virulent phages such as lytic phage (for instance, the λ phage of Escherichia coli), T4 phage, T7 phage and M13 phage may be cited. In addition, as bacteriophages infecting bacteria other than Escherichia coli, multiple bacteriophages have been reported, including, for instance, Phage K, which infects staphylococcus (Staphylococci) [Journal of Bacteriology 186, 2862-2871 (2004)], Mycobacteriophage, which infects Mycobacterium tuberculosis and the like, and these phages or phagemid vectors can be used appropriately depending on the target bacteria.

The therapeutic agent for infectious disease comprising as the active ingredient the gene encoding the antibody of the present invention or a fragment containing the antigen-binding site of the antibody may further contains substances that promotes gene introduction into bacteria, or medically acceptable additives. As substances that promote gene introduction into cell, cationic lipids, cationic polymers or hydrophobic polymers may be cited. As pharmaceutically acceptable additive or biocompatible materials may be cited. The above gene or vector may be carried by a biocompatible material. Concretely, a biocompatible material is collagen, gelatin or a mixture thereof.

### Example

The present invention is further described in detail in the following examples; however, the present invention is not to be limited to the scope of the examples.

Reference Example 1: Construction of the Escherichia coli MazF [MazF (E.coli)] expression plasmid vector, expression and purification of MazF (E.coli)
From the amino acid sequence of MazF from Escherichia coli (SEQ ID:1), according to the disclosures in the WO 2004/113498 pamphlet, DNA was constructed in which, from the gene sequence encoding MazF (E.coli), the ACA sequences were substituted with another nucleotide sequences without changing the amino acid residues encoded by the sequences. Note that, in order to carry out purification easily, a His-Tag sequence was added to the N-terminal end of MazF (E.coli), and the codons of the gene sequence encoding MazF (E.coli) were optimized, considering the codon usage frequency in Escherichia coli. A DNA fragment containing this DNA was chemically synthesized and inserted between the NcoI and HindIII sites of the pET21 (manufactured by Novagen) gene expression plasmid vector in which the ACA sequences in the gene sequence between the T7 promoter and terminator were substituted with other nucleotide sequences to prepare a recombinant plasmid, pETALHNMazF2.

Escherichia coli JM109 was transformed with this plasmid. After culturing one colony of the obtained transformant in a 5 mL LB medium at 37°C for 8 hours with shaking, the culture was expanded with 80 mL LB medium and the plasmid pETALHNMazF2 was purified from the cell body using the QIA filter Plasmid Maxi Kit (manufactured by Qiagen). Escherichia coli BL21 (DE3) was transformed with the obtained plasmid pETALHNMazF2, and the obtained transformant was Jar-cultured in 3 L LB medium.
The obtained cell body was suspended in PBS containing 400 mM NaCl and 10 mM imidazole, then sonicated to prepare a cell-free extract, and MazF (E.coli) was purified from this using His Trap HP (manufactured by GE health care).

Reference Example 2: Construction of the Escherichia coli MazE [MazE (E.coli)] expression plasmid vector, expression and purification of MazE (E.coli)
According to the disclosures in WO 2004/113498 pamphlet, a DNA fragment containing the DNA encoding a protein containing a His-Tag sequence and a Myc-Tag sequence at the N-terminal end of the amino acid sequence of MazE from Escherichia coli (SEQ ID:4) was chemically synthesized and inserted between the NdeI site and EcoRI site of a gene expression plasmid vector, pET21a (manufactured by Novagen), to prepare a recombinant plasmid, pHisMycMazE.

Escherichia coli JM109 was transformed with this plasmid. After culturing one colony of the obtained transformant in a 5 mL LB medium at 37°C for 8 hours with shaking, the culture was expanded with 80 mL LB medium and the plasmid pHisMycMazE was purified from the cell using QIA filter Plasmid Maxi Kit. Escherichia coli BL21 (DE3) was transformed with the obtained plasmid pHisMycMazE and the obtained transformant was Jar-cultured in 3 L LB medium. When OD 600 nm was close to 0.5, IPTG was added in such a way that the final concentration was 1 mM to induce expression, and the culture continued thereafter for 4 hours. The obtained cell body was suspended in PBS containing 400 mM NaCl and 10 mM imidazole, then sonicated to prepare a cell-free extract, and MazE (E.coli) was purified from this using His Trap HP.

Reference Example 3: Expression and purification of MazF [MazF (BCG)] and MazE [MazE (BCG)] from Mycobacterium bovis BCG
Construction of the expression plasmid vectors for MazF [MazF (BCG)] and MazE [MazE (BCG)] from Mycobacterium bovis BCG and protein expression/purification were carried out as follows. With the disclosures in WO 2007/013265 pamphlet, FEMS Microbiology Letter, Volume 274, pp73-82 (2007) and The Journal of Biological Chemistry, Volume 281, pp18638-18643 (2007) as references, a DNA containing the gene encoding MazF (BCG) (SEQ ID:7) was synthesized and digested with the restriction enzymes Nde I and Xho I. This DNA was cloned between the restriction enzyme sites Nde I-Xho I of the pET21a (Novagen) vector to construct a MazF (BCG) expression plasmid vector, pET-Mb2014c. In addition, a DNA containing the gene encoding MazE (BCG) (SEQ ID:8) was synthesized and this was digested with restriction enzymes Nde I and Hind III. This DNA was cloned between the restriction enzyme sites Nde I-Hind III of the pET21a (Novagen) vector to construct a MazE (BCG) expression plasmid vector, pHisMazEmb.

With these two plasmid vectors, Escherichia coli BL21 (DE3) was transformed with each, and the obtained transformants were Jar-cultured in 3 L LB medium. When OD 600 nm became close to 0.5, IPTG was added in such a way that the final concentration was 1 mM to induce expression, and cultivation was continued thereafter for 2 hours. The obtained cells were suspended in PBS containing 400 mM NaCl and 10 mM imidazole, then sonicated to prepare cell-free extracts, and MazF (BCG) and MazE (BCG) were purified using His Trap HP.
Note that, a TA system that is identical to MazF (BCG) and MazE (BCG) is present in Mycobacterium tuberculosis.

Reference Example 4: Expression and purification of MazF [MazF (S. aureus)] and MazE [MazE (S. aureus)] from Staphylococcus aureus
Construction of the expression plasmid vectors for MazF [MazF (S. aureus)] and MazE [MazE (S. aureus)] from Staphylococcus aureus, and protein expression/purification were carried out as follows. With the disclosures in Journal of Bacteriology, Volume 189, pp8871-8879 (2007) as reference, a DNA containing the gene encoding MazF (S. aureus) (SEQ ID:9) was synthesized and this was digested with the restriction enzymes Nde I and BamH I. This DNA was cloned between the restriction enzyme sites Nde I-BamH I of the pET28a (Novagen) vector to construct a MazF (S. aureus) expression plasmid vector, pET28mazFsa. In addition, a DNA containing the gene encoding MazE (S. aureus) (SEQ ID:10) was synthesized and digested with the restriction enzymes Nco I and BamH I. This DNA was cloned between the restriction enzyme sites Nco I-BamH I of the pET28a (Novagen) vector to construct a MazE (S. aureus) expression plasmid vector, pHisPrS2MazEsa. pHisPrS2MazEsa is an expression vector for a chimeric protein wherein two molecules of Protein S from Myxococcus xanthus are linked to the N-terminal end of MazE (S. aureus).

Escherichia coli BL21 (DE3) was transformed with these two plasmid vectors, and the obtained transformants were Jar-cultured in 3 L LB medium. When OD 600 nm was close to 0.5, IPTG was added in such a way that the final concentration was 1 mM to induce expression, and cultivation was continued thereafter for 4 hours. The obtained cells were suspended in PBS containing 400 mM NaCl and 10 mM imidazole, then sonicated to prepare cell-free extracts, and MazF (S. aureus) and MazE (S. aureus) were purified using His Trap HP.

### Example 1: Preparation of anti-MazF antibody and anti-MazE antibody

MazF (E.coli), MazE (E.coli), MazF (BCG), MazE (BCG), MazF (S. aureus) and MazE (S. aureus) prepared in Reference Examples 1 to 4 were prepared as antigen solutions at 1 mg/mL. Hereafter, in Example 1, they are sometimes described as MazE or MazF regardless of the origin of MazF or MazE. These antigen solutions were each administered to four BALB/c mice, and antisera and hybridomas were obtained according to methods disclosed in Methods in Enzymology, Volume 182 (1990) and Monoclonal Antibodies: Principles and Practice, Academic Press (1996). For this antiserum or hybridoma culture supernatant, ELISA was carried out using 96-well microplates in which MazF or MazE prepared in Reference Examples 1 to 4 were solid-phased according to the conventional methods, then, antibodies specifically binding to MazF or MazE were selected.

Anti-MazF antibodies and anti-MazE antibodies can also be obtained by screening antibody gene libraries. The antibody gene library can be prepared for instance with the peripheral blood lymphocytes of a healthy subject as materials, by amplifying and cloning into an appropriate vector the gene encoding the region from the antibody variable regions (VH, VL) to the constant regions (CH1, CL). Regarding methods for preparing an antibody gene library or a phage display library, it is possible to make reference to the laboratory manuals given below as well as original articles cited therein or the like.
[Methods In Molecular Biology Volume 248 "Antibody Engineering Methods and Protocols", Edited by Benny K.C. Lo, Humana Press (2004)]
["Phage Display, A Laboratory Manual", Edited by Carlos F. Barbas III, Dennis R. Burton, Jamie K. Scott, Gregg J. Silverman, Cold Spring Harbor Laboratory Press (2001)]

Anti-MazF (E.coli) antibodies 2C7, 21B-8H, 40-10A, 2H10, 2H8, 3B7 and 3B11, anti-MazF (S. aureus) antibodies 7B-7F and 8A-5E, and anti-MazF (BCG) antibodies 2A-7G and 11A-5D were obtained in this way, and polyclonal antibodies, monoclonal antibodies, Fab fragments of monoclonal antibodies and genes coding therefor used in the following examples were prepared appropriately.

By using plastic tubes on which MazF or MazE prepared in Reference Examples 1 to 4 has been solid-phased according to the conventional methods, and carrying out biopanning with the above documents and the like as references, Fab, scFv, Fd, light chain, VH, VL that bind specifically to MazF or MazE, as well as genes encoding these antibody fragments and the nucleotide sequence information thereof can be obtained.

### Example 2: Influence of antibodies on the ribonuclease activity of MazF (E.coli)

The ribonuclease activity of MazF (E.coli) was measured as follows: Tris-HCl buffer at the final concentration of 10 mM, pH 7.5, containing or not containing 75ng of MazF (E.coli) and 15 ng of MazE (E.coli) was preincubated at 37°C for 15 minutes, then, 100 pmol of the substrate oligonucleotide MazG30 (SEQ ID:11) and an antibody sample were added and reacted at 37°C for 30 minutes. The reaction was quenched by adding an equal amount of 95% formaldehyde containing 25mM EDTA. The reaction solution was analyzed by electrophoresis using a 15% acrylamide gel. As a result, when only the substrate MazG30 and MazF were added, the substrate MazG30 was degraded and a band was generated at a position corresponding to a 14mer polypeptide. When MazE and MazF were added to the substrate MazG30, degradation of the substrate MazG30 was inhibited and the band at the position corresponding to a 14mer polypeptide was decreased. In addition, when MazE, MazF and a Fab fragment of the anti-MazF (E.coli) monoclonal antibody obtained in Example 1 were added to the substrate MazG30, the substrate MazG30 was degraded and a band was generated at a position corresponding to a 14mer.

### Example 3: Method for high sensitive measurement of inhibition of MazE-MazF interaction by an antibody

In order to measure the ribonuclease activity of MazF with high sensitivity, an activity measurement system using a chimeric oligonucleotide labeled with a fluorescent substance and a quenching substance was constructed, taking reference to the following documents.

WO 2007/109781 pamphlet
Analytical Biochemistry, Volume 371, pp173-183 (2007)
Molecular Microbiology, Volume 69, pp559-569 (2008)
Journal of Bacteriology, Published online ahead of print on 27 February 2009

In this assay system, when the double-labeled chimeric oligonucleotide is cleaved by the ribonuclease activity of MazF, the distance between the fluorescent substance and the quenching substance becomes apart, generating fluorescence. When MazE is present with MazF in the activity measurement system, since the MazE-MazF complex is formed, the ribonuclease activity of MazF is suppressed and the substrate is not degraded; however, in case an antibody inhibiting the MazE-MazF interaction is present together in the reaction system, dissociation of the MazE-MazF complex occurs, and the ribonuclease activity of MazF is shown, so that the substrate is degraded and a fluorescence signal is observed.
As substrates for measuring the ribonuclease activity of MazF, fluorescently labeled chimeric oligonucleotides described in Table 1 were used with the above documents as references. In Table 1, rU represents a ribonucleotide (uracil).

**[Table 1]**

| MazF | Double-labeled chimeric oligonucleotide substrate | | SEQ ID |
|---|---|---|---|
| MazF (E.coli) | Substrate 1 | FAM-5'GATArUACATATG3'-Eclipse | 12 |
| MazF (BCG) | Substrate 2 | FAM-5'TTTCTrUCCTATGA3'-Eclipse | 13 |
| MazF(S.aureus) | Substrate 3 | FAM-5'TGACTrUACATCGAAG3'-Eclipse | 14 |

### Example 4: influence of anti-MazF (E.coli) antibody on MazE-MazF interaction

Measurement of the inhibitory activity of the anti-MazF (E.coli) antibody on the MazE (E.coli)-MazF (E.coli) interaction was carried out using the method of Example 3 as follows:
Using a microplate for fluorescence measurements, 10 µL of 1 µM MazF (E.coli) and 25 µL of antibody solution were mixed and reacted at 37°C for 15 minutes, then, 5µL of 1 µM MazE (E.coli) was added and reacted at 4°C for 60 minutes. Thereafter, 10 µL of substrate solution was added, reacted at 37°C for 90 minutes, and the fluorescence intensity at 485 nm excitation wavelength and 535 nm fluorescence wavelength was measured with a fluorescence plate reader (manufactured by Berthold). The composition of the substrate solution was 50mM Tris-HCl buffer pH 7.5 containing 312.5 nM Substrate 1, 16.75 U/mL RNase inhibitor (manufactured by Takara Bio Inc.), and complete, EDTA-free protease inhibitor cocktail (manufactured by Roche) at 2.5-fold concentration.

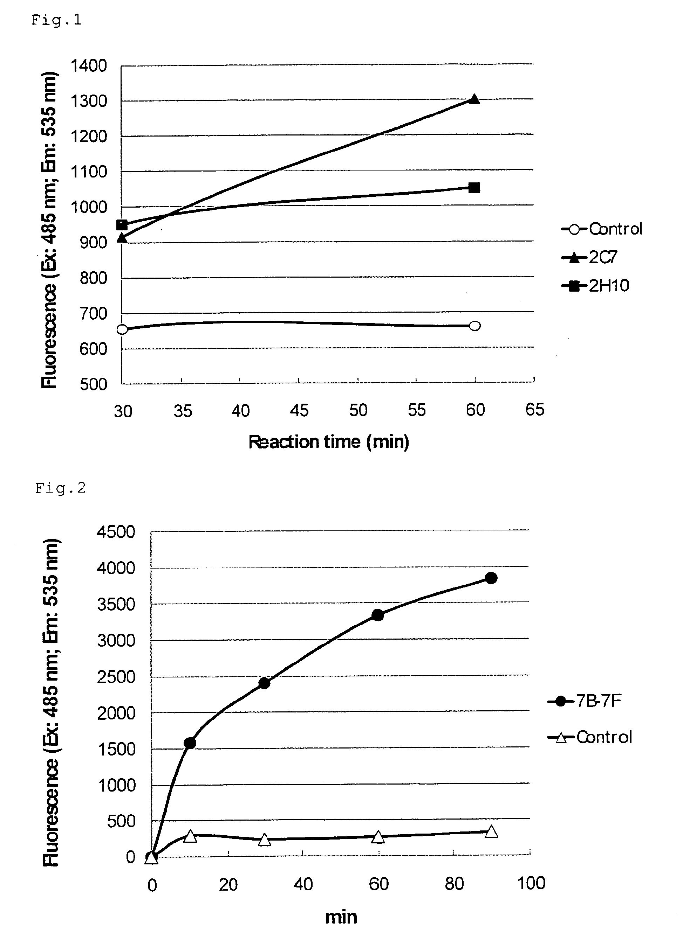

The result of using, as the antibody solution, one in which an H chain Fd fragments (VH-CH1) of the anti-MazF (E.coli) antibodies 2C7 and 2H10 prepared in Example 1 was proteosynthesized with a cell-free system using PROTEIOS (brand) Wheat germ cell-free protein synthesis kit (Toyobo), is shown in Fig. 1. As a control, one for which a protein synthesis reaction was carried out without adding the mRNA of the antibody Fd fragment was used.

When the control solution was added, no increase in fluorescence signal was observed, and the ribonuclease activity of MazF (E.coli) remained suppressed by MazE (E.coli); in contrast, but, when the synthesized Fd fragments of 2C7 and 2H10 were added, the substrate was degraded by the ribonuclease activity of MazF (E.coli) and the fluorescence signal was increased along with the reaction time.

### Example 5: Influence of anti-MazF (S. aureus) antibody on the MazE-MazF interaction

Measurement of the inhibitory activity of the antibody on the MazE (S. aureus)-MazF (S. aureus) interaction 3 was carried out using the method of Example as follows:
Using a microplate for fluorescence measurements, 10 µL of 0.5 µM MazF (S. aureus) and 25 µL of antibody solution (25 pmol) were mixed and reacted at 37°C for 15 minutes, then, 5 µL of 0.5 µM MazE (S. aureus) was added and reacted at 4°C for 60 minutes. Thereafter, 10 µL of substrate solution was added, reacted at 37°C for 90 minutes, and the fluorescence intensity at 485 nm excitation wavelength and 535 nm fluorescence wavelength was measured with a fluorescence plate reader. The composition of the substrate solution was 50mM Tris-HCl buffer pH 7.5 containing 312.5 nM Substrate 3, 16.75 U/mL RNase inhibitor, and complete, EDTA-free protease inhibitor cocktail at 2.5-fold concentration.

The result of using, as the antibody solution, an antibody purified from mouse ascites, which is the mouse anti-MazF (S. aureus) monoclonal antibody 7B-7F prepared in Example 1, is shown in Fig. 2. As control, 10mM Tris-HCl buffer pH 7.5 was used.

When the control solution was added, no increase in fluorescence signal was observed, and the ribonuclease activity of MazF (S. aureus) remained suppressed by MazE (S. aureus); in contrast, when the monoclonal antibody 7B-7F was added, the substrate was degraded by the ribonuclease activity of MazF (S. aureus) and the fluorescence signal was increased along with the reaction time.

### Example 6: Influence of anti-MazF (BCG) antibody on the MazE-MazF interaction

Measurement of the inhibitory activity of the antibody on the MazE (BCG)-MazF (BCG) interaction was carried out using the method of Example 3 as follows:
Using a microplate for fluorescence measurements, 10 µL of 4 µM MazF (BCG), 25µL of antibody solution (80 pmol) were mixed and reacted at 37°C for 15 minutes, then, 5 µL of 4 µM MazE (BCG) was added and reacted at 4°C for 60 minutes. Thereafter, 10 µL of substrate solution was added, reacted at 37°C for 90 minutes, and the fluorescence intensity at 485 nm excitation wavelength and 535 nm fluorescence wavelength was measured with a fluorescence plate reader. The composition of the substrate solution was 50mM Tris-HCl buffer pH 7.5 containing 312.5 nM Substrate 2 , 16.75 U/mL RNase inhibitor, and complete, EDTA-free protease inhibitor cocktail at 2.5-fold concentration.

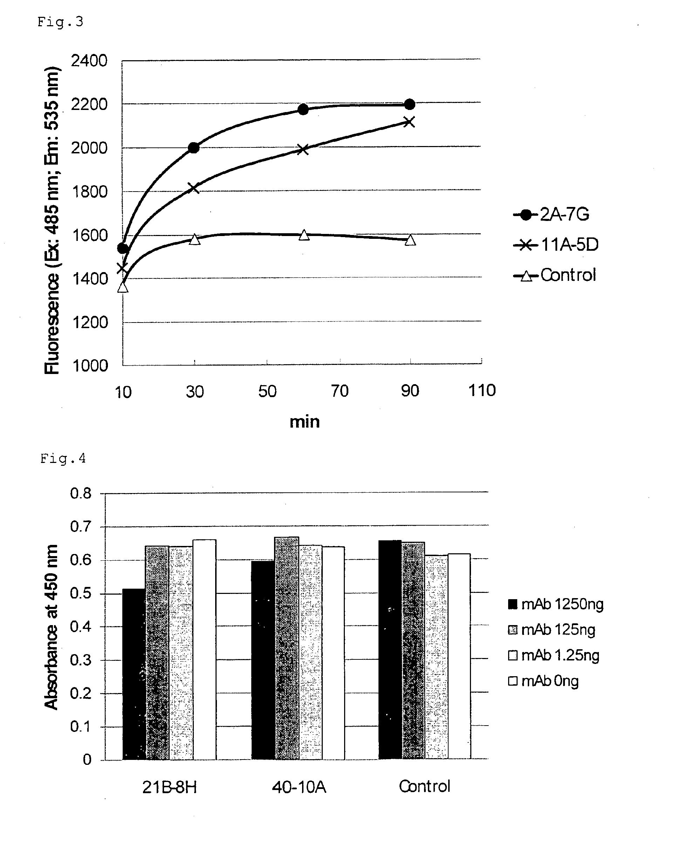

The result of using, as the antibody solution, antibodies purified from mouse ascites, which are the mouse anti-MazF (BCG) monoclonal antibodies 2A-7G and 11A-5D prepared in Example 1, is shown in Fig. 3. As control, 10mM Tris-HCl buffer pH 7.5 was used.

When the control solution was added, no increase in fluorescence signal was observed, and the ribonuclease activity of MazF (BCG) remained suppressed by MazE (BCG) ; in contrast, when the monoclonal antibody 2A-7G or 11A-5D was added, the substrate was degraded by the ribonuclease activity of MazF (BCG) and the fluorescence signal was increased along with the reaction time.

### Example 7: Influence of anti-MazF antibody on the MazE-MazF interaction

Measurement of the inhibitory activity of the antibody on the MazE-MazF interaction was carried out by a competition assay method using a solid-phased antigen plate. MazE or MazF from Escherichia coli or Staphylococcus aureus was solid-phased at a concentration of 2 µg/mL respectively in each well of a 96-well microplate manufactured by Nunc. Solid phasing was carried out at 4°C for 14 hours.

First, a competition assay was carried out, in which anti-MazF antibody was added with varying concentrations, to MazE interacting with solid-phased MazF. A solid-phased MazF plate was blocked with a PBS solution containing 0.1% Tween20 (manufactured by Sigma) and 1% BSA (manufactured by Sigma). A mixed solution of antibody prepared in Example 1 and MazE was added to the solid-phased MazF plate, incubated further at 4°C for 60 minutes, thereafter, the plate was washed with a PBS solution containing 0.1% Tween20. As control, a PBS solution containing 0.1% Tween 20 and 1% BSA, which is an antibody dilution solution, was used. In order to detect MazE bound on the solid phase plate, an antibody against Myc-Tag sequence [MazE (E.coli)] or ProteinS [MazE (S. aureus)] contained in MazE were used. An 2 µg/mL antibody solution for the reaction of the HRP-labeled anti-Myc-Tag sequence antibody and antibody solution at 0.2 µg/mL concentration for the reaction of the HRP-labeled anti-ProteinS antibody were added and reacted at room temperature for 60 minutes. After washing them with a PBS solution containing 0.1% Tween20, the substrate 3,3',5,5'-Tetramethylbenzidine (TMB, manufactured by Sigma) was added for coloring, and the reaction was quenched by adding an equal amount of 1N sulfuric acid. Thereafter, the absorption at 450 nm was measured with a microplate reader.

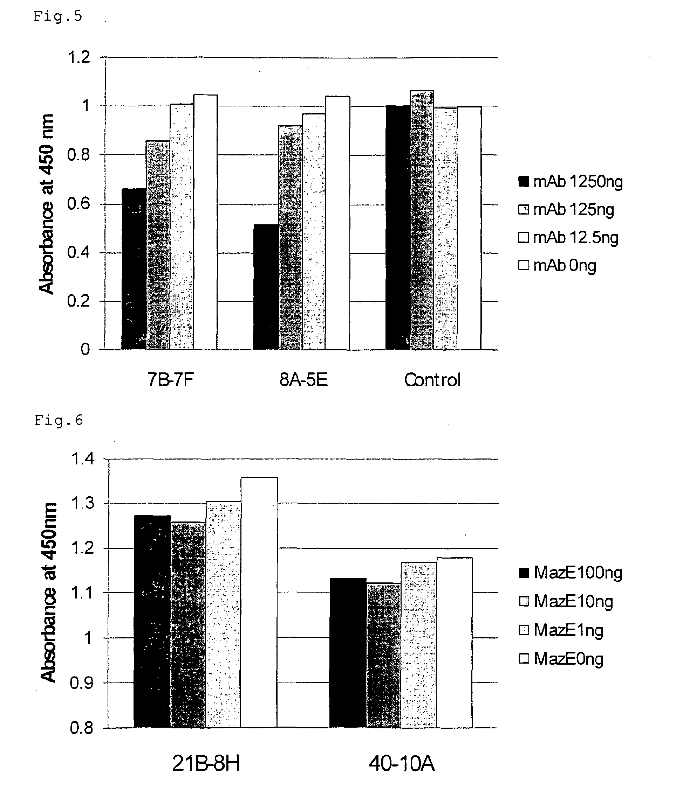

The result of inhibition of the MazE-MazF interaction by the anti-MazF (E.coli) monoclonal antibody 21B-8H or 40-10A prepared in Example 1 is shown in Fig. 4. When the antibody concentration was increased, the amount of MazE binding to the MazF solid-phased on the plate was decreased. On the other hand, there was no change in the MazE amount with the control.
The result of inhibition of the MazE-MazF interaction by the anti-MazF (S. aureus) monoclonal antibody 7B-7F or 8A-5E prepared in Example 1 is shown in Fig. 5. The amount of MazE binding to the MazF solid-phased on the plate was decreased in an antibody concentration-dependent manner. On the other hand, there was no change in the MazE amount with the control.

Next, a competition assay was carried out, in which MazE or MazF was added with varying concentrations. A solid-phased MazF plate was used upon evaluating the influence of an anti-MazF antibody and a solid-phased MazE plate was used upon evaluating that of an anti-MazE antibody. While the solid-phased plates were blocked with a PBS solution containing 0.1% Tween20 and 1% BSA, in the case of the solid-phased MazF (E.coli) plate only, blocking was carried out with a PBS solution containing BSA at 1%. A mixed solution of antibody and MazE or MazF was added to a solid-phased MazE or MazF plate, further incubated at 4°C for 60 minutes, thereafter, the plate was washed with a PBS solution containing 0.1% Tween20. The concentration of the antibody was 50 ng in the case of the anti-MazF (E.coli) antibody, 25 ng in the case of the anti-MazE (S. aureus) antibody and 25 ng in the case of the anti-MazF (S. aureus) antibody. The antibodies bound to the solid phase plates were detected by adding a HRP-labeled antimouse IgG antibody. The reaction for the secondary antibody was at room temperature for 60 minutes. After the plate was washed with a PBS solution containing 0.1% Tween20, TMB was added for coloring, and the reaction was quenched by adding an equal amount of 1N sulfuric acid. Thereafter, the absorption at 450 nm was measured with a microplate reader.

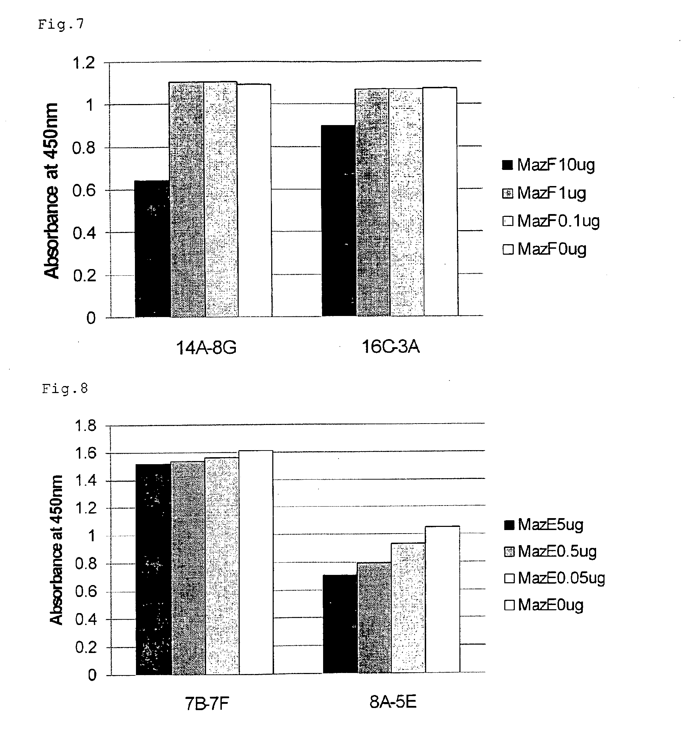

The result of inhibition of the MazE-MazF interaction by the anti-MazF (E.coli) monoclonal antibody 21B-8H or 40-10A prepared in Example 1 is shown in Fig. 6. When the amount of MazE was increased, the amounts of anti-MazF (E.coli) antibodies 21B-8H and 40-10A binding to MazF solid-phased on the plate was decreased depending on the amount of MazE added.
The result of inhibition of the MazE-MazF interaction by the anti-MazE (S. aureus) monoclonal antibody 14A-8G or 16C-3A prepared in Example 1 is shown in Fig. 7. The anti-MazE (S. aureus) monoclonal antibodies 14A-8G and 16C-3A binding to MazE solid-phased on the plate was decreased in a manner dependent on the amount of MazF added.
The result of inhibition of the MazE-MazF interaction by the anti-MazF (S. aureus) monoclonal antibody 7B-7F or 8A-5E prepared in Example 1 is shown in Fig. 8. The anti-MazF (S. aureus) monoclonal antibodies 7B-7F and 8A-5E binding to MazF solid-phased on the plate was decreased depending on the amount of MazE added.

### Example 8: Influence of anti-MazF antibody on Escherichia coli growth

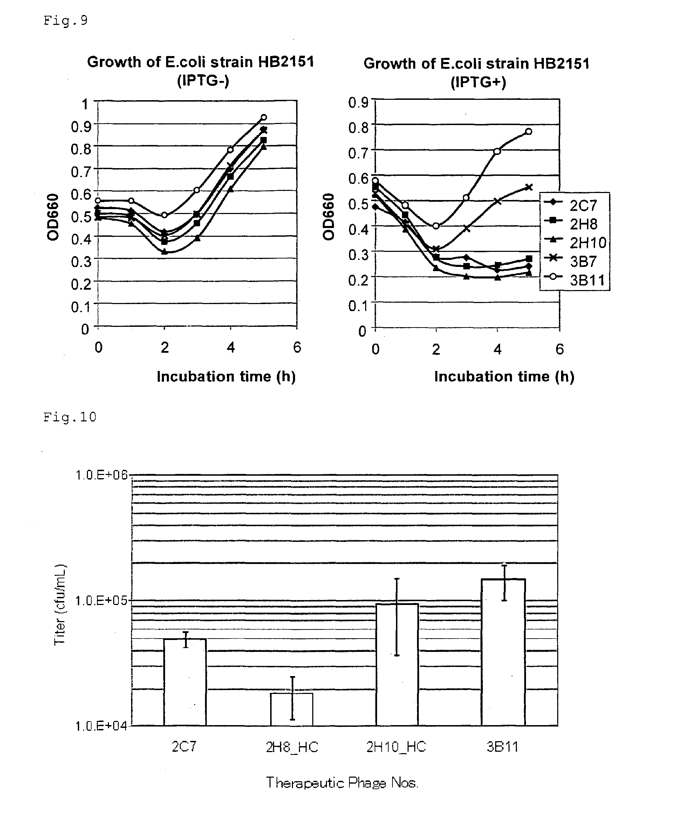

The gene encoding the H chain Fd fragment (VH-CH1) of the Fab fragments 2H10, 2H8, 3B7 and 3B11 from the anti-MazF (E.coli) antibody 2C7 prepared in Example 1 was inserted operably into the downstream of the Lac promoter of a phagemid vector to prepare an expression phagemid vector. This expression phagemid vector and M13KO7 helper phage were used to prepare therapeutic M13 phages (2C7, 2H10, 2H8) and control M13 phages 3B7 and 3B11. Escherichia coli HB2151 strain was transformed by infection with the prepared phages, and the expression of the Fab fragment gene or the Fd fragment gene was induced by adding IPTG to a final concentration of 1 mM and ampicillin to a final concentration of 100 µg/mL. The result of the growth curve of Escherichia coli is shown in Fig. 9. In samples in which the Fab fragment 2C7 or the H chain Fd fragments 2H10 and 2H8 were expressed, the growth of the host Escherichia coli was inhibited. On the other hand, when IPTG was not added, no growth inhibition of host Escherichia coli was observed in any samples.

Example 9: mouse model for the treatment of Escherichia coli infectious disease using anti-MazF antibody gene
The therapeutic M13 phages (2C7, 2H10 and 2H8) and the control M13 phage 3B11 used in Example 8 were prepared. Infection model experiments using mice were carried out as follows: Escherichia coli HB2151 strain was cultured with shaking overnight in 2mL of 2xYT culture medium at 37°C. The next morning, the culture solution was inoculated at 1% in 100mL of fresh 2xYT culture medium, cultured with shaking at 37°C for 1.5 hours, then the cells were harvested, 2xYT was added to the cell body and a cell concentration was adjusted to 5x108 cfu/mL. Into the peritoneal cavity of a 6-week old BALB/c mouse was administered 0.2mL of a dilute Escherichia coli solution thereof (1x108 cfu). Immediately thereafter, 0.2mL of a 5x108 cfu/mL therapeutic M13 phage solution or control M13 phage solution containing 125mM IPTG was administered into the peritoneal cavity on the side opposite to the side where the Escherichia coli solution was administered. After 5 hours, intraperitoneal cells were recovered with 7mL of PBS, colonies appearing after spreading this peritoneal cavity solution diluted with 2xYT on a 2xYT agar plate containing 100 µg/mL ampicillin and 2% glucose were counted to measure the viable cell count. The experimental results are shown in Fig. 10. In the group treated with the therapeutic M13 phage, the number of Escherichia coli in the peritoneal cavity of the mouse was decreased compared to the group treated with the control M13 phage 3B11.

### Industrial Applicability

From the above results, an antibody or a fragment containing the antigen-binding site of the antibody, showing the effect of suppressing pathogenic bacterial growth or killing bacteria by specifically binding to the toxin or the antitoxin and inhibiting the toxin-antitoxin interaction without suppressing the activity of the toxin in the bacterial TA system, and genes encoding them are provided. In addition, a therapeutic agent for infectious diseases containing as active ingredient these antibodies or fragments containing the antigen-binding site of the antibody, and a therapeutic agent for infectious diseases using genes encoding these active ingredients are provided, resulting in a therapeutic method against multiple drug-resistant bacteria, which is currently a serious problem.

### Sequence listing free text

SEQ ID NO: 1: Amino acid sequence of E.coli MazF.
SEQ ID NO: 2: Amino acid sequence of Mycobacterium tuberclosis MazF.
SEQ ID NO: 3: Amino acid sequence of Staphylococcus aureus MazF (S. aureus).
SEQ ID NO: 4: Amino acid sequence of E.coli MazE.
SEQ ID NO: 5: Amino acid sequence of Mycobacterium tuberclosis MazE.
SEQ ID NO: 6: Amino acid sequence of Staphylococcus aureus MazE (S. aureus).
SEQ ID NO: 7: Nucleic acid sequence containing MazF (BCG).
SEQ ID NO: 8: Nucleic acid sequence containing MazE (BCG).
SEQ ID NO: 9: Nucleic acid sequence containing MazF (S. aureus).
SEQ ID NO: 10: Nucleic acid sequence containing MazE (S. aureus).
SEQ ID NO: 11: Synthetic oligonucleotide MazG30.
SEQ ID NO: 12: Nucleic acid sequence of substrate-1.
SEQ ID NO: 13: Nucleic acid sequence of substrate-2.
SEQ ID NO: 14: Nucleic acid sequence of substrate-3.

## Claims

1. A prophylactic or therapeutic agent for a bacterial infectious disease comprising, as an active ingredient, an antibody binding to a toxin or an antitoxin constituting a bacterial toxin-antitoxin system to inhibit toxin-antitoxin interaction, or a gene thereof.

2. The prophylactic or therapeutic agent according to claim 1, wherein said antibody is a monoclonal antibody against a toxin or an antitoxin.

3. The prophylactic or therapeutic agent according to claim 2, wherein said antibody is a monoclonal antibody against a toxin or an antitoxin of a bacterium selected from the group consisting of Escherichia coli, Mycobacterium tuberculosis and Staphylococcus.

4. The prophylactic or therapeutic agent according to any one of claims 1 to 3, wherein said toxin-antitoxin system is a toxin-antitoxy system containing a toxin having a sequence-specific endoribonuclease activity.

5. The prophylactic or therapeutic agent according to any one of claims 1 to 4, wherein said antibody is a recombinant antibody.

6. The prophylactic or therapeutic agent according to any one of claims 1 to 5, wherein said antibody is a humanized antibody or a fully human antibody.

7. The prophylactic or therapeutic agent according to any one of claims 1 to 6, wherein said bacterial infectious disease is an infectious disease selected from the group consisting of tuberculosis, MRSA infectious disease and VRE infectious disease.

8. An inhibitory agent for weight loss in a bacterial infectious disease containing, as an active ingredient, an antibody binding to a toxin or an antitoxin constituting a bacterial toxin-antitoxin system to inhibit toxin-antitoxin interaction.
